# EUROPÄISCHE PATENTSCHRIFT

(11) **EP 0 283 925 B1**
(45) Veröffentlichungstag und Bekanntmachung des Hinweises auf die Patenterteilung: **11.12.1996**
(21) Anmeldenummer: 88104216.2
(22) Anmeldetag: 17.03.1988
(51) Int. Cl.: C08G 63/90, C08G 63/06

(54) **Verfahren zur Reinigung resorbierbarer Polyester**
Process for the purification of resorbable polyester
Procédé de purification de polyester résorbable

(30) Priorität: 19.03.1987 DE 3708916
(43) Veröffentlichungstag der Anmeldung: 28.09.1988
(73) Patentinhaber: BOEHRINGER INGELHEIM KG, D-55216 Ingelheim (DE); BOEHRINGER INGELHEIM INTERNATIONAL GmbH, D-55216 Ingelheim am Rhein (DE)
(72) Erfinder: Bendix, Dieter, Dr., Ingelheim am Rhein (DE); Reichert, Dieter, Dr., D-6507 Ingelheim am Rhein (DE); Schärfe, Michael, Dr., D-6200 Wiesbaden (DE)

(56) Entgegenhaltungen:
- WO-A-84/04311
- BE-A- 654 188
- DE-A- 2 419 330
- DE-A- 2 543 384
- FR-A- 2 086 047
- FR-A- 2 370 767
- US-A- 2 342 387
- US-A- 3 661 864
- US-A- 4 182 850
- PATENT ABSTRACTS OF JAPAN Band 11, Nr. 101 (C-413)(2548), 31. März 1987; & JP-A-61 252 228 (TORAY IND INC.) 10.11.1986

## Beschreibung

Die Erfindung betrifft ein Verfahren zur Reinigung von resorbierbaren Polyestern.

Die Herstellung und Verwendung von resorbierbaren Polyestern ist aus dem Stand der Technik bekannt, so z.B. aus den DE-OS'en 12 93 396, 15 95 085, 17 20 211, 21 18 127, 22 06 144, 25 01 448, 27 00 729, 28 25 911, 28 27 289, 28 50 824, den europäischen Patentanmeldungen 73 655, 98 394, 102 265, 108 933, 176 895 u.a..

Wie aus zahlreichen Publikationen bekannt ist, liegt der große Vorteil resorbierbarer Polyester, insbesondere derer auf der Basis von Milch- oder Glykolsäure, in der Tatsache, daß sie im menschlichen oder tierischen Gewebe oder in menschlichen oder tierischen Flüssigkeiten vollständig zu körpereigenen Verbindungen abgebaut werden, wobei die Abbaurate des Polymeren, je nach Verwendungszweck, von wenigen Stunden bis zu mehreren Monaten variiert werden kann. Die Abbauprodukte gelangen in den normalen biochemischen Stoffwechsel und werden entweder direkt ausgeschieden oder letztlich zu Wasser und Kohlendioxid metabolisiert.

In den letzten Jahren hat das Interesse an der Verwendung resorbierbarer Polyester sehr stark zugenommen, so z.B. im Bereich der Chirurgie als Nahtmaterial oder Klammern und im Bereich der Osteosynthese als Ersatz für Metallimplantate.

Von besonderem Interesse sind die Anwendungen resorbierbarer Polyester ferner im Bereich galenischer Zubereitungen mit verzögerter Wirkstofffreigabe zur Herstellung von Depotformen und Implantaten.

Durch Variation der Monomeren und der Comomeren, der molaren Verhältnisse, der Reaktionsbedingungen, der Art und Menge eines oder mehrer Katalysatoren, sowie durch den Zusatz von Regulatoren, gelingt es, für viele Anwendungsgebiete die optimalen Polyester herzustellen. Aufgrund der spezifischen Verwendung dieser resorbierbaren Polyester in menschlichen oder tierischen Körpern ist es notwendig oder zumindest wünschenswert, nur solche Polyester zu verwenden, die frei von Verunreinigungen sind, die möglicherweise Irritationen auslösen können. Zu solchen Verunreinigungen zählen beispielsweise nicht umgesetzte Restmonomere, Molekulargewichtsregulatoren und Polymerisationskatalysatoren.

Die physikalische Struktur der Polyester kann kristallin, teilkristallin oder völlig amorph sein.

Die Kristallinität von Polyestern kann nach verschiedenen Kriterien beurteilt werden (s. z.B. US-PS 4 523 591, US-PS 3 878 284), vorzugsweise jedoch durch die Aufnahme von DSC-Kurven (differential scanning calorimetry). Dabei versteht man unter teilkristallinen oder amorphen, resorbierbaren Polyestern solche, die im DSC nur einen Glasübergang zeigen. Kristalline Polyester zeigen, unter Umständen erst nach einer entsprechenden Vorbehandlung, zusätzlich Rekristallisations- und Schmelzpeaks.

Teilkristalline oder amorphe, resorbierbare Polyester sind besonders für den Bereich der kontrollierten Wirkstofffreigabe interessant, da sie kürzere Freigabezeiten und einen schnelleren Abbau im Körper als die kristallinen Polyester zeigen.

Solche teilkristalline oder amorphe, resorbierbare Polyester können durch Lösungs-, Emulsions- oder Substanzpolymerisation in der Schmelze hergestellt werden. Die so erhaltenen Polyester müssen zur Entfernung von Restmonomeren, Oligomeren, Zusätzen zur Molekulargewichtsregulation, Katalysatoren und sonstigen von der Polymerisationsreaktion herrührenden, Verunreinigungen gereinigt werden.

Beispielsweise erfolgt die Reinigung bei Substanzpolymerisaten durch Auskochen der gemahlenen Polyester mit einem geeigneten Lösungsmittel (US-PS Nr. 4 523 591). Üblicherweise werden Polymerisate zur Abtrennung der oben genannten Verunreinigungen in einem geeigneten organischen Lösungsmittel gelöst, gereinigt, etwa durch Filtration mit Aktivkohlezusatz und durch Zugabe eines Fällungsmittels wieder zurückgewonnen.

Im Gegensatz zu kristallinen Polyestern können teilkristalline oder amorphe Polyester nicht aus ihren Lösungen in einem organischen Lösungsmittel durch Zugabe eines Fällungsmittels isoliert werden.

Versucht man etwa, durch Zugabe eines Fällungsmittels oder aber durch Eintragen der Lösung in ein Fällungsmittel das Polymere zu isolieren, so erhält man wegen des amorphen Aufbaus des Polymeren nur eine gallertartige, große Mengen Fällungs- und Lösungsmittel einschließende Masse, die nicht weiterverarbeitet werden kann. Ebensowenig gelingt bei teilkristallinen oder amorphen Polyestern eine Reinigung durch Umkristallisation aus einem Lösungsmittel oder einem Lösungsmittelgemisch.

Es ist die Aufgabe der vorliegenden Erfindung ein Verfahren zur Reinigung von resorbierbaren Polyestern, mit einer Kristallinität von bis zu 20% zur Verfügung zu stellen.

Erfindungsgemäß werden teilkristalline oder amorphe Polyester dadurch gereinigt, daß man das Polymer in einem geeigneten Lösungsmittel oder Lösungsmittelgemisch löst, anschließend die Polymerlösung so mit einem Fällungsmittel in Kontakt bringt, daß das dabei ausfallende Polymer infolge der auftretenden Scherkräfte in kleinste Teilchen zerteilt wird, wobei das Scherfeld durch eine Vorrichtung erzeugt wird, die aus einer Zweistoffdüse und aus einem mit einem Fällungsmittel gefüllten Behälter besteht, in den die Zweistoffdüse hineinragt.

Es können selbstverständlich mit dieser Methode auch kristalline Polyester vorteilhaft gereinigt werden, jedoch stehen hier auch die oben erwähnten Methoden zur Verfügung.

In der DE-PS 26 46 332 ist beispielsweise die Herstellung von Fibrillen aus fluorhaltigen Polymerisaten mit Hilfe von Dispergiermaschinen oder Zweistoffdüsen, in der DE-OS 2 208 921 die Herstellung von Kurzfasern aus thermoplastischen Kunststoffen durch Fällung unter Einfluß von Scherkräften beschrieben.

Die WO-A 84 04 311 beschreibt ein Verfahren zur Entfernung von nicht abreagiertem Monomer und Katalysator aus bioresorbierbaren Lactid/Caprolactam Copolymeren durch Lösen und anschließendes Ausfällen des Polymers aus der Lösung (Anspruch 1; Seite 13, Zeile 5 bis Seite 14, Zeile 6; Seite 17, Beispiel I, Zeilen 17 - 29; Seite 19, Beispiel 11, Zeilen 13 - 26). Gemäß den Beispielen I und II wird das Rohpolymer in Methylenchlorid gelöst und der in einem Behälter befindlichen Lösung dann zum Wiederausfällen des Copolymers unter heftigem Rühren mittels eines mechanischen Rührers Isopropanol zugegeben. Dabei wird eine Polymermasse erhalten, die zerstückelt und erst bei tiefen Temperaturen zu einem Pulver vermahlen werden kann.

Daneben hat sich die DE-A-2 543 383 die Aufgabe gestellt, ein wirtschaftliches Verfahren zur Herstellung von Polyamidimid-Pulvern bereitzustellen, da sich zur Verarbeitung durch Preßsintern eignet. Diese Aufgabe wird durch die Ausfällung des Polyamidimids aus seinen konzentrierten Lösungen in einem turbulenten Scherfeld gelöst (Anspruch 1; Seite 2, Absätze 1 - 3). Die Abbildung auf Seite 7 dieser Offenlegungsschrift illustriert eine bevorzugte Ausführungsform, die sich einer Strahldüse mit einer zentralen Bohrung für die Polymerlösung und einem dazu konzentrischen Ringkanal für das Fällungsmittel bedient (Seite 5, 3. Absatz). Gemäß Beispiel 1 treten Lösung und Fällungsmittel mit großer Geschwindigkeitsdifferenz aus der Düse aus und passieren unter Ausbildung eines turbulenten Scherfelds ein Umpulsaustauschrohr, das in einem umgebenden Behälter gegenüber der Düse angeordnet ist. Das Polymer fällt dabei teilchenförmig aus (Beispiel 1 : 2 - 3 mm lang, 0,5 mm dick; Beispiel 2 : 3 - 7 mm lang, 1 mm dick).

Des weiteren wird in der DE-A 2 419 330 ein Verfahren zur Herstellung von fließbaren Überzugspulvern aus Polymerharzen beschrieben, die sich zur Aufbringung mittels der Wirbelbett-Technologie eignen. Dabei wird eine Polymerisatlösung zu einem Nebel versprüht, dessen Tröpfchen dann mit einem Ausfällungsmittel in Berührung gebracht werden, wobei Harzteilchen feiner Teilchengröße ausfallen (Anspruch 1, Seite 1, Zeile 9 bis Seite 3, letzter Absatz, Zeile 4). Das Ausfällungsmittel kann in einem gerührten Reaktor enthalten sein, als fallender Flüssigkeitsfilm vorliegen, oder auch seinerseits als Sprühnebel vorliegen (Seite 10, ab Zeile 3). Unter den geeigneten Polymerharzen sind auch Polyester angeführt (Seite 5, Zeile 3), allerdings keine (bio)resorbierbaren.

Es wurde nun überraschenderweise gefunden, daß resorbierbare Polyester mit einer Kristallinität bis zu 20 %, die nach dem erfindungsgemäßen Verfahren aus ihren Lösungen aufgearbeitet werden, praktisch frei von Verunreinigungen, wie z. B. Katalysatoren, Restmonomeren etc., sind. Geeignete Polyester im Sinne der Erfindung sind solche, die in einem organischen Lösungsmittel oder in einem Lösungsmittelgemisch, gegebenenfalls nach geeigneter Vorbehandlung, z.B. Tempern, löslich sind. Die Kristallinität der Polyester liegt zwischen null und zwanzig Prozent.

Bevorzugt sind Homo- und Copolymere auf der Basis von Hydroxycarbonsäuren, wie beispielsweise Polymerisate von Glycolid, Lactid, Methylglycolid, Dimethylglycolid, Polymethylglycolid, Diethylglycolid, Dibutylglycolid, Caprolacton, Valerolacton, Decalacton, Propiolacton, Butyrolacton, Pivalolacton, sowie Polymere auf der Basis von Trioxanon (1,3 und 1,4), Dioxanon (1,3 und 1,4) substituiertem Dioxanon, Trimethylencarbonat, Ethylencarbonat und Propylencarbonat.

Als weitere Comonomere sind u.a. die folgenden Verbindungen geeignet:
Milchsäure, Glykolsäure, Pentaerythrit, Sorbit, Adonit, Xylit, Fructose, Epichlorhydrin, Isopropylmorpholin, Isopropylmethylmorpholindion, β-Propiolsäure, Tetramethylglycolid, β-Butyrolacton, γ-Butyrolacton, Pivalolacton, α-Hydroxybuttersäure, α-Hydroxyisobuttersäure., α-Hydroxyvaleriansäure, α-Hydroxyisovaleriansäure, α-Hydroxycapronsäure, α-Hydroxyisocapronsäure, α-Hydroxy-α-ethylbuttersäure, α-Hydroxy-α-methylvaleriansäure, α-Hydroxyheptansäure, α-Hydroxyoctansäure, α-Hydroxydecansäure, α-Hydroxytetradecansäure, α-Hydroxystearinsäure.

Besonders bevorzugt sind Homopolymere aus Lactid, Copolymere aus verschiedenen Lactiden und Copolymere aus Lactiden und Glycolid mit inhärenten Viskositäten zwischen 0.1 und 10 dl/g.

Als Lactid können L-, D-, meso- oder D,L-Lactid oder Gemische davon eingesetzt werden.

Bevorzugt sind weiterhin Homopolymere aus D,L-Lactid mit einer inhärenten Viskosität ovn 0.1 bis mindestens 6 dl/g, entsprechend mittleren Molekulargewichten von 2000 bis mindestens 2 Mio, berechnet aus der inhärenten Viskosität durch Approximation des Staudinger Indexes nach Solomon und Ciuta (Lit. J. Appl. Polym. Sci, 6,24 (1962), 683-6) und Einsetzen in die Mark-Houwink-Gleichung mit den Paramtern K = 6.06 E-4 und a = 0.64 (Lit. J. Rak, J.L. Ford, Ch. Rostron u. V. Walter, Pharm. Acta Helv., 60, 5-6 (1985), 162-9).

Ferner sind bevorzugt Copolymere aus L-Lactid und Glycolid mit inhärenten Viskositäten bis mindestens 4.5 dl/g. Daraus berechnen sich nach der oben angegebenen Methode und den Mark-Houwink-Parametern K = 5.45 E-4 und a = 0.73 (Lit. S. Gogolewski u. A. J. Pennings, J.Appl. Polym. Sci., 28, 1045-61) mittlere Molekulargewichte von 50 000 bis mindestens 250 000.

Bevorzugt sind weiterhin Copolymere aus D,L-Lactid und Glycolid mit inhärenten Viskositäten von mindestens 2.5 dl/g, entsprechend mittleren Molekulargewichten von mindestens 110 000, berechnet mit den obigen Mark-Houwink-Parametern für Copolymere; weiterhin Copolymere aus ε-Caprolacton mit L-, D,L-Lactid und Glycolid und inhärenten Viskositäten bis mindestens 4 dl/g; ferner
Poly(L-lactid-co-D,L-lactid) im Verhältnis 9 : 1, Poly(L-lactid-co-glycolid) im Verhältnis 7 : 3, Poly(D,L-lactid-co-glycolid) im Verhältnis 3 : 1, Poly(D,L-lactid-co-glycolid) im Verhältnis 1 : 1 Poly(D,L-lactid-co-glycolid) im Verhältnis 45:55.
(Die Angaben beziehen sich auf Mol-Verhältnisse).

Beispielsweise werden Homopolymere und Copolymere der Milch- und Glykolsäure vorzugsweise durch ringöffnende Polymerisation der cyclischen Diester der Milch- und Glykolsäure, nämlich des Lactids und Glycolids, hergestellt.

Auf Grund der Chiralität der Milchsäure können in die ringöffnende Polymerisation die optisch aktiven L- und D-Lactide, sowie das optisch inaktive meso-Lactid und das Racemat D,L-Lactid eingesetzt werden.

Die Bedingungen dieser Polymerisation sind bekannt. Die Herstellung der Homopolymeren erfolgt in Lösung, Emulsion oder in der Schmelze. Die Copolymere jeder beliebigen Zusammensetzung werden wegen der unterschiedlichen Reaktivität der Comonomeren in Emulsion, vorzugsweise jedoch durch Substanzpolymerisation in der Schmelze hergestellt. Bei der Polymerisation läßt isch durch Variation der Reaktionsparameter Temperatur, Zeit und Katalysatorkonzentration, sowie durch Zugabe eines oder mehrerer Co-Katalysatoren das jeweils gewünschte Molekulargewicht und die entsprechende Molekulargewichtsverteilung einstellen.

Zur Durchführung des Reinigungsverfahrens werden die nach bekannten Verfahren hergestellten kristallinen, teilkristallinen oder amorphen Polyester in einem geeigneten organischen Lösungsmittel oder einem Gemisch verschiedener organischer Lösungsmittel gelöst.Als Lösungsmittel sind alle die organischen Lösungsmittel geeignet, die den entsprechenden Polyester ausreichend gut lösen und mit dem gewählten Fällungsmittel im betrachteten Mischungsverhältnis mischbar sind.

Vorzugsweise werden Aceton, 1,4-Dioxan, Dimethylacetamid, Tetrahydrofuran, Toluol, Dimethylformamid, Dimethylsulfoxid oder chlorierte Kohlenwasserstoffe, wie z.B. Chloroform oder Methylenchlorid, als Lösungsmittel eingesetzt.

Wenn es erforderlich erscheint, können der eigentlichen Fällung nach zusätzliche Reinigungsschritte, wie z.B. Filtrieren unter Aktivkohlezusatz, vorgeschaltet werden. Dann wird der Polyester aus der Polymerlösung in Gegenwart eines Fällungsmittels unter Ausbildung eines Scherfeldes durch eine Dispergiereinheit gefällt. Voraussetzung dafür ist, daß sich Lösungs- und Fällungsmittel im Gebiet der betrachteten Zusammensetzung vollständig miteinander mischen.

Als Fällungsmittel kann z.B. Wasser eingesetzt werden, dem zur Erreichung spezieller, zusätzlicher Reinigungseffekte geringe Mengen einer anorganischen oder organischen Säure, einer anorganischen oder organischen Base, Mittel zur Veränderung der Oberflächenspannung oder eines Komplexbildners zugesetzt werden können. Als Fällungsmittel sind ferner Methanol, Ethanol, Frigene, Kohlenwasserstoffe und Kohlenwasserstoffgemische wie z.B. Petrolether, geeignet, sowie organische Lösungsmittel, die ein Nichtlösungsmittel für den Polyester sind.

Vorteilhaft ist es, wenn das Fällungsmittel einen höheren Siedepunkt und eine niedrigere Flüchtigkeit als das Lösungsmittel besitzt.

Wird als Lösungsmittel Chloroform oder Methylenchlorid verwendet, sind die bevorzugten Fällungsmittel Hexan, Petrolether oder ein Frigen.

Wird als Lösungsmittel Aceton, Dimethylacetamid, Dioxan, Tetrahydrofuran, Dimethylformamid oder Dimethylsulfoxid bzw. deren Gemische verwendet, ist das bevorzugte Fällungsmittel Wasser.
Im allgemeinen beträgt die Temperatur der beteiligten Komponenten zwischen -20°C und der Siedepunkt des Lösungsmittelgemisches, bevorzugt zwischen -20°C und Raumtemperatur.

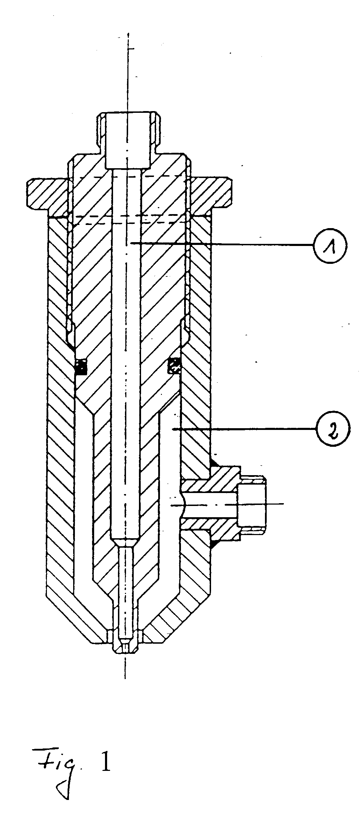

Das erfindungsgemäße Verfahrenarbeitet mit einer Zweistoffdüse (s. Fig. 1). Dabei wird die Polymerlösung aus einem unter Druck stehenden Behälter durch die innere Bohrung (1) der Düse gedrückt, während das Fällungsmittel mit hoher Geschwindigkeit durch den äußeren, konzentrisch angeordneten Ringkanal (2) gepumpt wird.
Unmittelbar nach dem Austritt aus der Düse kommt es zur Verwirbelung des Zentralstrahls (Polymerlösung) mit dem in Form eines Hohlkegels austretenden konzentrischen Strahls (Fällungsmittel). Durch die hohen Geschwindigkeitsunterschiede der beiden Medien und der dadurch auftretenden Scherwirkung kommt es zu einer Dispergierung der Polymerlösung im Fällungsmittel. Durch die Anordnung der Düse am Boden eines senkrecht stehenden, mit Fällungsmittel gefüllten Zylinders können die ausgefällten Polymerfibrillen in dem hohen Überschuß an Fällungsmittel aufschwimmen und abgeführt werden.

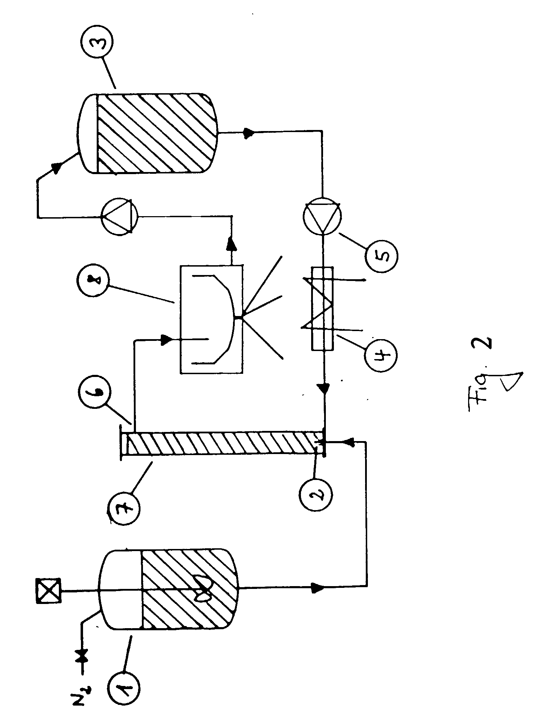

Eine diese Düse benutzende, kontinuierlich arbeitende Vorrichtung zeigt Fig. 2.
Dabei wird die Polymerlösung aus einem Rührwerksapparat (1) durch Anlegen eines Stickstoffüberdrucks durch die innere Bohrung der Düse (2) gepreßt, während gleichzeitig aus einem Vorratsbehälter (3) über einen Wärmeaustauscher (4) das Fällungsmittel mit einer Pumpe (5) durch den äußeren Ringkanal der Düse gepumpt wird. Über den Auslauf (6) des senkrecht stehenden Fällungszylinders (7) wird das Polymer/Fällungsmittelgemisch einer mit niedriger Drehzahl laufenden Zentrifuge (8) zugeführt. Das ablaufende Fällungsmittel wird in den Vorratsbehälter (3) zurückgepumpt. Da sich bei dieser Vorgehensweise das Lösungsmittel im Fällungsmittel anreichert, muß bei einer für das jeweilige Lösungsmittel/Fällungsmittelsystem zu ermittelnden Konzentration des Lösungsmittels das Fällungsmittel ausgetauscht werden. Das Lösungsmittel kann bei Bedarf, z.B. durch Destillation, zurückgewonnen werden.

Statt der Zentrifuge kann auch eine Nutsche (Drucknutsche), ein Aufgußfilter, ein kontinuierlich arbeitendes Bandfilter oder eine kontinuierlich arbeitende Schneckenpresse eingesetzt werden.

Die so gewonnenen oder resorbierbaren Polyester mit einer Kristallinität von bis zu 20% liegen nach dem Trocknen in fädriger bis filzartiger Fibrillenform vor und können durch Vermahlen unter Zusatz eines Kältemittels wie Trockeneis oder flüssigen Stickstoff homogenisiert werden.

Die erfindungsgemäß gereinigten Polyester eignen sich besonders zur Herstellung von Gegenständen, die im menschlichen oder tierischen Körper resorbiert werden können.

Hierzu zählen insbesondere Gegenstände zur Osteosynthese und Arzneistoffträger. Letztere können in Form von Tabletten oder Kapseln, aber auch als implantierbare oder injizierbare Depotformen vorliegen.

Im folgenden werden stellvertretende einige Produkte zur medizinischen Verwendung genannt, die vorteilhaft aus resorbierbaren Polymeren hergestellt werden können.

Aus resorbierbaren Polymeren hergestellte Produkte:
1. Massive Produkte, formgepreßt oder maschinell bearbeitet:
   Orthopädische Dübel, Klammern, Schrauben und Platten, Clips (z.B. für vena cava),
   Krampen,
   Haken, Knöpfe oder Schnapper,
   Knochenersatz (z.B. Kieferprothese),
   Nadeln,
   Nichtpermanente Intrauterineinsätze (antispermizid),
   Temporäre Drainage- oder Prüfschläuche oder Kapillaren,
   Chirurgische Instrumente,
   Gefäßimplantate oder -stützmittel,
   Wirbelscheiben,
   extrakorporale Schläuche für Nieren und Herz-Lungenmaschinen.
   Langsam aufschließbares Ionenaustauscherharz,
   langsam aufschließbare, Wirkstoffe freigebende Erzeugnisse (Pillen, Pellets),
   verstärkte Knochendübel, Nadeln usw. Implantierbare mit Arzneistoffen beladene Pellets,
   Stifte, Folien und sonstige Formkörper zur kontrollierten Wirkstofffreigabe.
2. Faserprodukte, gestrickt oder gewebt,
   einschließlich Velour,
   Brandverbände,
   Bruchkissen,
   Absorbierendes Papier oder Tupfer,
   Medizinalverbände,
   Gesichtsplastiken,
   Gazen, Gewebe, Tuch, Filz oder Schwamm zur Hämostase,
   Gaze-Bandagen,
   Dentalpackungen,
   Nahtmaterial, einschließlich Ligaturen. Arterientransplantat oder -ersatz,
   Bandagen für Hautoberflächen,
   Brandverbände (in Kombination mit anderen Polymerfilmen).
3. Pulverfömige Produkte, hergestellt durch Sprühtrocknung, Mahlung oder Mikroverkapselung.
   Injizierbares oder implantierbares mit Arzneistoffen beladenes Pulver zur kontrollierten und verzögerten Freigabe des Wirkstoffes.
   Mikroporöse Formkörper, Folien, Puder, Granulate zur Beladung mit Wirkstoffen.
4. Verschiedenes
   Flocken oder Puder für Verbrennungen oder Abschürfungen,
   Schaumstoff als absorbierbare Prothese,
   Ersatz für Draht beim Schienen,
   Filmspray für prothetische Elemente und zur Wundversorgung.

### Beispiel

### Beispiel 1

100 g eines Poly(D,L-lactid-co-glycolid) 50: 50 Mol.%-Rohproduktes werden in 1 l Aceton gelöst. Die Lösung wird in einen Druckbehälter überführt und der Inhalt des Behälters mit einem Überdruck von ca. 3 bar durch den inneren Kanal (Druchmesser 0.7 mm) einer Zweistoffdüse gepreßt. Gleichzeitig wird mit einer Pumpe Wasser mit einem Durchsatz von ca. 50 l/min durch das äußere Segment der Zweistoffdüse gepumpt. Beide Strahlen treten in einen senkrecht stehenden, mit Fällungsmittel gefüllten Zylinder (Fällungsrohr) aus, wobei durch die dann eintretende Scherung und Verwirbelung die Dispergierung der Polymerlösung und ein Ausfällen des Polymeren erreicht wird.

Der Ablauf des Fällungsrohrs führt in einen mit Wasser gefüllten 100 l-Randkessel mit Doppelmantelkühlung. Das aufschwimmende Polymere kann an der Wasseroberfläche mit einem Sieb abgetrennt werden. Das noch sehr viel Fällungsmittel und damit in geringen Mengen auch noch Lösungsmittel enthaltende Polymere wird mit kaltem Wasser nachgewaschen, kräftig abgesaugt und im Umlufttrockenschrank bei 40 Grad Celsius getrocknet. Das Fällungsmittel wird vom Boden des Randkessels wieder abgesaugt und der Zweistoffdüse wieder zugeführt. Die gesamte Polymermenge kann ohne Wechsel des Fällungsbades aufgearbeitet werden. Acetonkonzentration im Wasser nach der Fällung: 1,5 %).
Ausbeute: 93,8 g, 94 % des Einsatzes
Inhärente Viskosität: 2.08 dl/g (Chloroform, 25 Grad Celsius)
Wassergehalt: 0.37 % nach Karl-Fischer-Titration

### Beispiel 2

1.730 kg eines Poly(D,L-lactid-co-glycolid) 75:25 Mol %-Rohproduktes werden in 17 1 Aceton gelöst. Die Lösung wird in ein Druckfilter überführt und mit einem Stickstoffüberdruck von ca. 3 bar durch den inneren Kanal der Zweistoffdüse gepreßt. Gleichzeitig wird mit einer Pumpe das Fällungsmittel Wasser mit einem Durchsatz von ca. 50 l/min durch das äußere Segment der Zweistoffdüse gepumpt.
Beide Strahlen treten in einen senkrecht stehenden, mit Fällungsmittel gefüllten Zylinder aus, wobei durch die dann eintretende Scherung und Verwirbelung die Dispergierung der Polymerlösung und ein Ausfällen des Polymeren erreicht wird. Der Überlauf des Fällungsrohrs führt in ein drucklos gehaltenes Druckfilter, wobei das Wasser durch den hydrostatischen Druck wieder in einen darunter stehenden Auffangbehälter abfließt. Von hier wird es durch ein als Wärmeaustauscher ausgebildetes Rohr unter Kühlung mit Sole wieder durch die Zweistoffdüse gepumpt.

Nach Beendigung der Fällung wird das Druckfilter mit Stickstoff beaufschlagt und das Restwasser gut abgepreßt, das Polymere mit kaltem Wasser nachgewaschen und anschließend nochmals gut abgepreßt. Das Polymere wird dann im Umlufttrockenschrank bei 40 Grad Celsius getrocknet.
Ausbeute: 1.68 kg, 97 % des Einsatzes

Das getrocknete Polymere wird mit Trockeneis versetzt auf einer Alexanderreibe mit einem 0.6 mm Schräglochsieb gemahlen und dann im Umlufttrockenschrank bei 40 Grad Celcius getrocknet.
Ausbeute: 1.64 kg, 98 % des Einsatzes
Inhärente Viskosität: 0.68 dl/g (Chloroform, 25 Grad Celsius)
Wassergehalt: 0.43 % nach Karl-Fischer-Titration
Restmonomer: < 1 Gew.%

### Beispiel 3

0.770 kg eines Poly(D,L-lactid)-Rohproduktes werden in 20 1 1,4-Dioxan gelöst. Die Polymerlösung wird wie im Beispiel 2 beschrieben aufgearbeitet. Das noch wasserfeuchte Produkt wird mit Trockeneis versetzt und nach dem Durchfrieren über eine vorgekühlte Condux-Schneidmühle gemahlen.
Ausbeute: 0.70 kg, 78 % des Einsatzes
Inhärente Viskosität: 3.2 dl/g (Chloroform, 25 Grad Celsius)
Wassergehalt: 0.35 % nach Karl-Fischer-Titration
Restmonomere: nicht nachweisbar
Sn-Gehalt: 34 ppm (Einsatz 138 ppm Katalysator)

### Beispiel 4

24.8 kg eines Poly(D,L-lactid)-Rohproduktes werden in einem 250 l-Rührwerksapparat in ca. 240 1 Aceton gelöst. Der Inhalt des Apparates wird batchweise in einen 60 l-Druckfilter überführt und wie im Beispiel 2 beschrieben mit Wasser als Fällungsmittel gefällt. Der Über lauf des Fällungsrohrs wird diesmals einer langsam laufenden Zentrifuge zugeführt. Das ablaufende Wasser wird, wie beschrieben rezirkuliert.
Nach Beendigung der Fällung wird die Tourenzahl der Zentrifuge erhöht, das anhaftende Fällungsmittel möglichst weitgehend abgeschleudert, das Polymere auf der Schleuder dreimal mit kaltem Wasser gewaschen und nach dem Ausräumen der Schleuder im Umlufttrockenschrank bei 40 Grad Celsius getrocknet. Anschließend wurde das Polymere unter Zusatz von Trockeneis durch Mahlen über eine Alexanderreibe homogenisiert.
Ausbeute: 21.6 kg, 87 % des Einsatzes
Inhärente Viskosität: 0.93 dl/g (Chloroform, 25 Grad Celsius)
Wassergehalt: 0.36 % nach Karl-Fischer-Titration
Restmonomer: nicht nachweisbar

### Beispiel 5

Jeweils ca. 4 kg eines Poly(D,L-lactid-co-glycolid) 50:50 Mol%-Rohprodukts wurden entsprechend Beispiel 2 in Dioxan gelöst und mit Hilfe der Zweistoffdüse aus Wasser umgefällt.

| Ansatz Nr. | Restmonomer-Gehalt (Gew. %) | | Sn-Gehalt (ppm) | |
|---|---|---|---|---|
| | Rohprodukt | Endprodukt | Rohprodukt | Endprodukt |
| 1 | 5.3 | <0.1 | 75.5 | nicht nachweisbar |
| 2 | 3.8 | <0.1 | 75.5 | 9 |
| 3 | 2.7 | <0.1 | 50 | 3 |
| 4 | 12.4 | <0.1 | 25 | nicht nachweisbar |

### Beispiel 6

4 kg eines Poly(D,L-lactid-co-glycolid) 75:25 Mol%-Rohprodukts wurden entsprechend Beispiel 2 in Dioxan gelöst und mit Hilfe der Zweistoffdüse aus Wasser umgefällt.

| Restmonomer-Gehalt (Gew. %) | | Sn-Gehalt (ppm) | |
|---|---|---|---|
| Rohprodukt | Endprodukt | Rohprodukt | Endprodukt |
| 3.7 | <0.1 | 137.5 | 11 |

## Patentansprüche

1. Verfahren zur Reinigung von resorbierbaren Polyestern mit einer Kristallinität bis zu 20 %, dadurch gekennzeichnet, daß man das Polymer in einem Lösungsmittel löst, anschließend die Polymerlösung mit einem Fällungsmittel unter Einwirkung von hohen Scherkräften in einem turbulenten Scherfeld in innigen Kontakt bringt, wobei das turbulente Scherfeld durch eine Vorrichtung erzeugt wird, die aus einer Zweistoffdüse und aus einem mit Fällungsmittel gefüllten Behälter besteht, in welchen die Zweistoffdüse hineinragt, so daß das ausfallende Polymer in kleinste Teilchen zerteilt wird

2. Verfahren nach Anspruch 1, dadurch gekennzeichnet, daß es sich bei dem resorbierbaren Polyester um ein Polymer oder Copolymer auf der Basis von L-Lactid, D-Lactid, D,L-Lactid, meso-Lactid, Glycolid und/oder Caprolacton handelt.

3. Verfahren nach Anspruch 1 - 2, dadurch gekennzeichnet, daß dem Fällungsmittel ein Komplexbildner, eine Säure oder eine Base oder ein Hilfsstoff zur Veränderung der Oberflächenspannung zugesetzt wird.

4. Verfahren nach Anspruch 1 - 3, dadurch gekennzeichnet, daß das Lösungsmittel Aceton, Dimethylacetamid, Dioxan, Tetrahydrofuran, Dimethylformamid oder Dimethylsulfoxid und das Fällungsmittel Wasser ist, oder das Lösungsmittel Chloroform oder Methylenchlorid und das Fällungsmittel Hexan, Petrolether oder ein Frigen ist.

5. Verfahren nach einem der Ansprüche 1 bis 4, dadurch gekennzeichnet, daß der resorbierbare Polyester aus der Gruppe der Homo- und Copolymere auf der Basis von Hydroxycarbonsäuren, wie beispielsweise Polymerisate von Glykolid, Lactid, Methylglycolid, Dimethylglykolid, Polymethylglycolid, Diethylglycolid, Dibutylglycolid, Caprolacton, Valerolacton, Decalacton, Propiolacton, Butyrolacton, Pivalolacton, sowie Polymere auf der Basis von Trioxanon (1,3 und 1,4), Dioxanon (1,3 und 1,4) substituiertem Dioxanon, Trimethylencarbonat, Ethylencarbonat und Propylencarbonat ausgewählt ist und gegebenenfalls als Comonomer ein oder mehrere Verbindungen ausgewählt aus der Gruppe Milchsäure, Glykolsäure, Pentaerythrit, Sorbit, Adonit, Xylit, Fructose, Epichlorhydrin, Isopropylmorpholin, Isopropylmethylmorpholindion, β-Propionsäure, Tetramethylgycolid, β-Butyrolacton, γ-Butyrolacton, Pivalolacton, α-Hydroxybuttersäure, αHydroxyisobuttersäure, α-Hydroxyisovaleriansäure, α-Hydroxycapronsäure, α-Hydroxyisocapronsäure, α-Hydroxy- α-ethylbuttersäure, α-Hydroxy- α-methylvaleriansäure, α-Hydroxyheptansäure, α-Hydroxyoctansaure, α-Hydroxydecansäure, α-Hydroxytetradecansäure, α-Hydroxystearinsäure, α-Hydroxyvaleriansäure enthält.

6. Verfahren nach Anspruch 5, dadurch gekennzeichnet, daß der resorbierbare Polyester aus der Gruppe eines Copolymeren aus D,L-Lactid und Glycolid mit inhärenten Viskositäten von mindestens 2.5 dl/g, entsprechend mittleren Molekulargewichten von mindestens 110 000, eines Copolymeren aus ε-Caprolacton mit L-, D,L-Lactid und Glykolid und inhärenten Viskositäten bis mindestens 4 dl/g; Poly(L-lactid-co-D,L-Lactid) im Verhältnis 9 : 1, Poly(L-lactid-co-glycolid) im Verhältnis 7 : 3, Poly(D,L-lactid-co-glycolid) im Verhältnis 3 : 1. Poly(D,L-lactid-co-glycolid) im Verhältnis 1 : 1 und Poly(D,L-lactid-coglycolid) im Verhältnis 45 : 55 ausgewählt ist.

## Claims

1. Process for the purification of resorbable polyesters with a crystallinity of up to 20%, characterised in that the polymer is dissolved in a solvent, the polymer solution is subsequently brought into intimate contact with a precipitation agent under the effect of high shear forces in a turbulent shear field, the turbulent shear field being produced by an apparatus consisting of a two component nozzle and a container filled with precipitation agent into which the two component nozzle projects so that the polymer precipitated is divided up into minute particles.

2. Process according to claim 1, characterised in that the resorbable polyester is a polymer or copolymer based on L-lactide, D-lactide, D,L-lactide, meso-lactide, glycolide and/or caprolactone.

3. Process according to claims 1 to 2, characterised in that a complexing agent, an acid or a base or an adjuvant for altering the surface tension is added to the precipitation agent.

4. Process according to claims 1 to 3, characterised in that the solvent is acetone, dimethylacetamide, dioxan, tetrahydrofuran, dimethylformamide or dimethylsulphoxide and the precipitation agent is water, or the solvent is chloroform or methylene chloride and the precipitation agent is hexane, petroleum ether or a freon.

5. Process according to one of claims 1 to 4, characterised in that the resorbable polyester is selected from the group comprising the homopolymers and copolymers based on hydroxycarboxylic acids, such as, for example, polymers of glycolide, lactide, methylglycolide, dimethylglycolide, polymethylglycolide, diethylglycolide, dibutylglycolide, caprolactone, valerolactone, decalactone, propiolactone, butyrolactone, pivalolactone, and polymers based on trioxanone (1,3 and 1,4), dioxanone (1,3 and 1,4), substituted dioxanone, trimethylene carbonate, ethylene carbonate and propylene carbonate and optionally contains as comonomer one or more compounds selected from the group comprising lactic acid, glycolic acid, pentaerythritol, sorbitol, adonitol, xylitol, fructose, epichlorohydrin, isopropylmorpholine, isopropylmethylmorpholinedione, β-propionic acid, tetramethylglycolide, β-butyrolactone, γ-butyrolactone, pivalolactone, α-hydroxybutyric acid, α-hydroxyisobutyric acid, α-hydroxyisovaleric acid, α-hydroxycaproic acid, α-hydroxyisocaproic acid, α-hydroxy-α-ethylbutyric acid, α-hydroxy-α-methylvaleric acid, α-hydroxyheptanoic acid, α-hydroxyoctanoic acid, α-hydroxydecanoic acid, α-hydroxytetradecanoic acid, α-hydroxystearic acid and α-hydroxyvaleric acid.

6. Process according to claim 5, characterised in that the resorbable polyester is selected from the group of a copolymer of D,L-lactide and glycolide with inherent viscosities of at least 2.5 dl/g, corresponding to average molecular weights of at least 110,000, a copolymer of ε-caprolactone with L-, D,L-lactide and glycolide and inherent viscosities of up to at least 4 dl/g;
poly(L-lactide-co-D,L-lactide) in the ratio 9:1, poly(L-lactide-co-glycolide) in the ratio 7:3, poly(D,L-lactide-co-glycolide) in the ratio 3:1, poly(D,L-lactide-co-glycolide) in the ratio 1:1 and poly(D,L-lactide-co-glycolide) in the ratio 45:55.

## Revendications

1. Procédé de purification de polyesters résorbables ayant une cristallinité pouvant atteindre 20%, caractérisé en ce que l'on dissout le polymère dans un solvant, puis on met la solution de polymère en contact intime avec un précipitant sous l'action de forces de cisaillement importantes dans un champ de cisaillement turbulent, le champ de cisaillement turbulent étant produit par un dispositif qui consiste en une buse à deux substances et en un récipient rempli de précipitant dans lequel pénètre la buse à deux substances, de sorte que le polymère qui précipite est divisé en particules extrêmement petites.

2. Procédé selon la revendication 1, caractérisé en ce qu'il s'agit, concernant le polyester résorbable, d'un polymère ou copolymère à base de L-lactide, de D-lactide, de D,L-lactide, de méso-lactide, de glycolide et/ou de caprolactone.

3. Procédé selon les revendications 1 - 2, caractérisé en ce qu'au précipitant est ajouté un complexant, un acide ou une base ou une substance auxiliaire pour modifier la tension superficielle.

4. Procédé selon les revendications 1 - 3, caractérisé en ce que le solvant est l'acétone, le diméthylacétamide, le dioxane, le tétrahydrofurane, le diméthylformamide ou le diméthylsulfoxyde et le précipitant est l'eau, ou le solvant est le chloroforme ou le chlorure de méthylène et le précipitant est l'hexane, l'éther de pétrole ou un Fréon.

5. Procédé selon l'une des revendications 1 à 4, caractérisé en ce que le polyester résorbable est choisi dans le groupe des homo- et copolymères à base d'acides hydroxycarboxyliques comme par exemple les produits de polymérisation de glycolide, lactide, méthylglycolide, diméthylglycolide, polyméthylglycolide, diéthylglycolide, dibutylglycolide, caprolactone, valérolactone, décalactone, propiolactone, butyrolactone, pivalolactone, et des polymères à base de trioxanone (1,3 et 1,4), dioxanone (1,3 et 1,4), dioxanone substituée, carbonate de triméthylène, carbonate d'éthylène et carbonate de propylène et contient éventuellement comme comonomère un ou plusieurs composés choisis dans le groupe de l'acide lactique, de l'acide glycolique, du pentaérythritol, du sorbitol, de l'adonitol, du xylitol, du fructose, de l'épichlorhydrine, de l'isopropylmorpholine, de l'isopropylméthylmorpholinedione, de l'acide β-propionique, du tétraméthylglycolide, de la β-butyrolactone, de la γ-butyrolactone, de la pivalolactone, de l'acide α-hydroxybutyrique, de l'acide α-hydroxyisobutyrique, de l'acide α-hydroxyisovalérique, de l'acide α-hydroxycaproïque, de l'acide α-hydroxyisocaproïque, de l'acide α-hydroxy-α-éthylbutyrique, de l'acide α-hydroxy-α-méthylvalérique, de l'acide α-hydroxyheptanoïque, de l'acide α-hydroxyoctanoïque, de l'acide α-hydroxydécanoïque, de l'acide α-hydroxytétradécanoïque, de l'acide α-hydroxystéarique, de l'acide α-hydroxyvalérique.

6. Procédé selon la revendication 5, caractérisé en ce que le polyester résorbable est choisi dans le groupe d'un copolymère de D,L-lactide et de glycolide ayant des viscosités inhérentes d'au moins 2,5 dl/g, ce qui correspond à des masses moléculaires moyennes d'au moins 110000, d'un copolymère d'ε-caprolactone et de L-, D,L-lactide et de glycolide ayant des viscosités inhérentes atteignant au moins 4 dl/g, d'un copolymère (L-lactide-D,L-lactide) dans le rapport 9:1, d'un copolymère (L-lactide-glycolide) dans le rapport 7:3, d'un copolymère (D,L-lactide-glycolide) dans le rapport 3:1, d'un copolymère (D,L-lactide-glycolide) dans le rapport 1:1 et d'un copolymère (D,L-lactide-glycolide) dans le rapport 45:55.
